# EUROPEAN PATENT APPLICATION

(11) **EP 3 888 625 A1**
(43) Date of publication of application: **06.10.2021**
(21) Application number: 19888525.3
(22) Date of filing: 26.11.2019
(51) Int. Cl.: A61K 8/65, A61K 8/49, A61K 31/506, A61K 35/614, A61K 38/39, A61P 17/14, A61P 43/00, A61Q 7/00

(54) **COMPOSITION FOR SCALP AND HAIR**

(30) Priority: 27.11.2018 JP 2018221432
(71) Applicant: Jellyfish Research Laboratories, Inc., Yokohama-shi Kanagawa 230-0046 (JP)
(72) Inventor: KIHIRA Kouji, Yokohama-shi, Kanagawa 230-0046 (JP); BABA Takayuki, Yokohama-shi, Kanagawa 230-0046 (JP)
(74) Representative: J A Kemp LLP
(86) International application number: PCT/JP2019/046121
(87) International publication number: WO 2020/111047

(57) **Abstract**

It is an object of the present invention to discover a substance having the effect of enhancing the activity of minoxidil sulfotransferase in the scalp, and then, to use this substance together with minoxidil to provide a drug, in which the hair growth/hair restoration effect of the minoxidil is enhanced.

The present invention provides: a minoxidil sulfotransferase activity enhancer, comprising a jellyfish collagen as an active ingredient; and a composition for scalp and hair, comprising a jellyfish collagen and minoxidil or a derivative thereof.

## Description

### Technical Field

The present invention relates to a minoxidil sulfotransferase activity enhancer and a composition for scalp and hair, each comprising a jellyfish collagen as an active ingredient.

### Background Art

In recent years, hair trouble such as thinning hair or fallen hair has been increased due to various factors such as aging, stress and ultraviolet rays. The hair is an extremely important element for beauty, and thus, thinning hair or fallen hair exhibits negative effects on psychological aspects. Moreover, although thinning hair and fallen hair have been considered to be male worries, such thinning hair and fallen hair have also become serious problems for women due to a reduction in female hormone and the like. The development of hair care agents and the market therefor are increasing steadily.

Conventionally, a cause of androgenetic alopecia (AGA) has been considered to be the overaction of male hormones. In particular, it has been known that the main body of male hormone activity in organs such as hair roots or sebaceous glands is 5α-dihydrotestosterone generated as a result of the reduction of testosterone by testosterone-5α-reductase. As such, there have been proposed many hair restorers comprising a natural substance, etc. having an action to inhibit testosterone-5α-reductase (Patent Literatures 1 and 2, etc.).

Meanwhile, minoxidil (chemical name: 2,4-diamino-6-piperidinopyrimidine-3-oxide) has been developed as a hypotensive agent based on its vasodilator action. However, it was found that minoxidil causes a hirsutism phenomenon as a side effect. From such findings, minoxidil has started to be used as a drug that exhibits an excellent hair growth/hair restoration effect when it is externally applied. External agents comprising minoxidil have been commercially available both in Japan and abroad. Differing from the above-described testosterone-5α-reductase inhibitory action, minoxidil directly acts on hair follicles that envelop hair roots in the dermis and generates various growth factors, and thus, minoxidil has excellent hair growth phase-extending action and excellent hair growth phase-inducing action (Non Patent Literature 1, etc.).

Moreover, for the purpose of the reinforcement of the hair growth/hair restoration effect of minoxidil, alleviation of the skin irritancy side effects, stability in a solution, etc., hair restorers comprising other components in combination with the minoxidil have also been developed. For example, a hair restorer composition characterized by comprising minoxidil and crude drugs, namely, a Jatoba extract and/or a Betula extract (Patent Literature 3), a hair restorer comprising minoxidil and a plant extract from soybeans, pueraria roots or the like (Patent Literature 4), a hair restorer composition characterized by comprising minoxidil and carpronium chloride (Patent Literature 5), and the like have been reported. Thus, minoxidil has excellent hair growth/hair restoration effects, and there has been a high demand for hair care products comprising such minoxidil. On the other hand, it has also been reported that, since minoxidil exhibits its medicinal effects by being activated with sulfotransferase in a living body, the effects of the minoxidil are low when the sulfotransferase activity is low in the scalp (Non Patent Literature 2).

### Citation List

### Patent Literature

Patent Literature 1: Patent Publication (Kokai) No. 2012-229167 A
Patent Literature 2: Patent Publication (Kokai) No. 2012-176921 A
Patent Literature 3: Patent Publication (Kokai) No. 9-87148 A (1997)
Patent Literature 4: Patent Publication (Kokai) No. 2005-119996 A
Patent Literature 5: Patent Publication (Kokai) No. 2008-56703 A

### Non Patent Literature

Non Patent Literature 1: Messenger AG, Rundegren J, Minoxidil: mechanisms of action on hair growth, Br J Dermatol, 150: 186-94, 2004
Non Patent Literature 2: Hamamoto T, Mori Y, Res CommunChem Pathol Pharmacol. 66(1): 33-44. 1989

### Summary of Invention

### Technical Problem

Accordingly, it is an object of the present invention to discover a substance having the effect of enhancing the activity of minoxidil sulfotransferase in the scalp, and then, to use this substance together with minoxidil to provide a drug, in which the hair growth/hair restoration effect of the minoxidil is enhanced.

### Solution to Problem

As a result of intensive studies directed towards achieving the aforementioned object, the present inventors have found that a jellyfish collagen can enhance the activity of minoxidil sulfotransferase to convert minoxidil to minoxidil sulfate. In addition, a gene expression analysis was performed on human epidermal keratinocytes, to which jellyfish collagen had been added. As a result, a significant fluctuation (increase or decrease) was confirmed in terms of the expression level of a group of genes associated with the formation of hair, such as hair growth, cell proliferation, hair cycle, and hair follicle formation. Thus, it was found that the hair growth/hair restoration effect of minoxidil can be further improved, if a jellyfish collagen is used in combination with the minoxidil. The present invention has been completed based on such findings.

Specifically, the present invention encompasses the following inventions.
(1) A minoxidil sulfotransferase activity enhancer, comprising a jellyfish collagen as an active ingredient.
(2) An agent for promoting the expression of one or two or more types of hair growth/hair restoration-related factors selected from the group consisting of BMP2, BMPR1A, GSK3B, BCL2, VEGFB, VEGFC, FGF7, NOG, SMAD4, TGFB1, GAS1, THBS2, BMP7, EFNA1, IRF6, and SOX9, wherein the agent comprises a jellyfish collagen as an active ingredient.
(3) An agent for suppressing the expression of ILIA, comprising a jellyfish collagen as an active ingredient.
(4) A composition for scalp and hair, comprising a jellyfish collagen and minoxidil or a derivative thereof.
(5) The composition for scalp and hair according to the above (4), wherein the content ratio between the jellyfish collagen and the minoxidil or a derivative thereof is 1 : 10 to 1 : 10000 at a mass ratio.
(6) The composition for scalp and hair according to the above (4) or (5), which is in the form of a cosmetic, a quasi-drug, or a pharmaceutical product
(7) A beauty treatment method, comprising applying the composition for scalp and hair according to any one of the above (4) to (6) to scalp or hair.

The present application claims priority from Japanese Patent Application No. 2018-221432, filed on November 27, 2018; the disclosure of the description of which is hereby incorporated by reference.

### Advantageous Effects of Invention

Since a jellyfish collagen is capable of reinforcing the activity of minoxidil sulfotransferase in the scalp, it can enhance the hair growth/hair restoration effect of minoxidil by being used in combination with the minoxidil. Therefore, the composition for scalp and hair of the present invention comprising a jellyfish collagen is effective for the prevention and/or improvement of thinning hair or fallen hair.

### Description of Embodiments

Hereafter, the present invention will be described in detail.

### 1. Minoxidil sulfotransferase activity enhancer

The minoxidil sulfotransferase activity enhancer of the present invention comprises a jellyfish-derived collagen (hereinafter referred to as a "jellyfish collagen") as an active ingredient. Herein, the minoxidil sulfotransferase includes SULT1A1 (sulfotransferase family, cytosolic, 1A, phenol-preferring, member 1), SULT1A2 (sulfotransferase family, cytosolic, 1A, phenol-preferring, member 2), and SULT1A3 (sulfotransferase family, cytosolic, 1A, phenol-preferring, member 3).

The jellyfish collagen used in the present invention is typically a water-soluble undenatured collagen derived from a jellyfish. However, as long as the present jellyfish collagen has the above-described effects, it includes a denatured collagen subjected to a heat denaturation treatment and a low-molecular-weight collagen peptide prepared by decomposing these collagens by an enzyme treatment or an acidic treatment. In addition, a jellyfish extract comprising a jellyfish collagen may be directly used as a jellyfish collagen in the present invention, but such a jellyfish extract is preferably isolated and/or purified by concentration, liquid-liquid distribution, or various types of chromatography.

In the present invention, the term "jellyfish" is used to mean jellyfish belonging to Cnidaria, and examples of the jellyfish may include moon jellyfish, red jellyfish, *Owan* jellyfish, *Nomura's* jellyfish, *Andon* jellyfish, *Bizen* jellyfish, and *Habu* jellyfish. The jellyfish collagen used in the present invention is not particularly limited, as long as it is a collagen prepared from a jellyfish. The present jellyfish collagen is preferably derived from moon jellyfish, *Nomura's* jellyfish and *Bizen* jellyfish, since the safety of these types of jellyfishes to humans and other animals has been confirmed.

The jellyfish collagen used in the present invention can be prepared by a method comprising treating a jellyfish with a buffer or an aqueous solution having a salt concentration similar to marine water, and then extracting a jellyfish collagen from the treated solution. The site of the jellyfish used in the extraction is not particularly limited, and examples of the site of the jellyfish that can be used in the extraction may include epidermis, oral arms, corpus ventriculi, a body fluid, and a liquid component generated as a result of cryopreservation or preservation at normal temperature. The extraction can be carried out, for example, by treating a jellyfish with a phosphate buffer or a solution containing approximately 0.2% sodium chloride, and then leaving the treated solution at a low temperature of approximately 4°C overnight. The extracted jellyfish collagen is subjected to a treatment such as freeze-drying or spray drying, so that water can be removed from the extracted jellyfish collagen. Otherwise, an aqueous solution comprising a jellyfish collagen is subjected to salting-out using inorganic salts such as ammonium sulfate, or to a precipitation method using alcohol such as ethanol, so as to precipitate the jellyfish collagen, and a method such as centrifugation or filtration is then applied, so that the jellyfish collagen can be separated. A method of obtaining an extract comprising a jellyfish collagen using the aforementioned jellyfish sites as materials, and a method of purifying the obtained extract according to a method commonly used in purification of proteins, such as ethanol precipitation or liquid chromatography, may be carried out in accordance with the descriptions of Japanese Patent No. 5775221 (WO2014/030323) and Patent Publication (Kokai) No. 2013-27381 A.

For example, the method described in Japanese Patent No. 5775221 is a fractional extraction method of jellyfish mucin and collagen, and this method comprises: (i) a protein separation step of mixing a liquid component obtained by solid-liquid separation of a hydrous crushed product obtained by crushing a jellyfish with water-soluble alcohol containing 1 to 4 carbon atoms (preferably, ethyl alcohol and propyl alcohol) so that the concentration of the water-soluble alcohol containing 1 to 4 carbon atoms becomes at least 50% by volume with respect to the entire mixture, and then subjecting the obtained mixture to solid-liquid separation to obtain a solid; and (ii) a step of mixing the solid obtained in the above-described protein separation step with an aqueous solution of water-soluble alcohol containing 1 to 4 carbon atoms (preferably, ethyl alcohol and propyl alcohol) having an alcohol concentration of 10% to 40% by mass, and then performing a solid-liquid separation operation on the obtained mixture, so that a mucin-containing liquid component and a collagen-containing solid can be obtained, separately. Subsequently, the obtained collagen-containing solid is subjected to a known purification operation such as, for example, ion exchange chromatography, and then, to a drying operation such as, for example, freeze-drying, so that the collagen can be isolated. The jellyfish collagen can be identified by quantifying the content of glycine (Gly) according to an amino acid analysis.

The jellyfish collagen prepared by the above-described method has a structure similar to the V-shaped collagen of mammals that is formed with the heterotrimer 1α2α3. When compared with collagens derived from other species, the jellyfish collagen is characterized in that it contains a large amount of sugar and also contains a large amount of hydroxylysine compared with hydroxyproline, and also in that it has an amino acid composition in which glycine (Gly) accounts for approximately one third of the total amino acid amount.

In addition, the jellyfish collagen used in the present invention is commercially available, and JelliCollagen (registered trademark) can be preferably used.

The jellyfish collagen prepared by the above-described method, etc. can be processed into any shapes such as powders, granules, and pellets, or it can be dissolved in a suitable solvent to prepare a solution. The solvent used to dissolve the jellyfish collagen is not particularly limited, and examples of the solvent that can be used herein may include: solvents generally used in a neutral range, such as water, a normal saline, or a phosphate buffer; and acidic solvents such as hydrochloric acid or acetic acid. When the jellyfish collagen is processed into powders, it is preferably preserved at -20°C to 10°C, and is further preferably preserved together with a desiccant such as silica gel. On the other hand, when the jellyfish collagen is processed into a solution, it is preferably preserved at 10°C or lower.

Upon the use of the jellyfish collagen in the agent of the present invention, it is preferable that the above-described jellyfish collagen and a jellyfish extract comprising the same have previously been subjected to a treatment of removing or reducing pathogenic components including endotoxin, antigenic components, and allergenic components from the jellyfish collagen and the jellyfish extract.

The jellyfish collagen has an action to reinforce minoxidil sulfotransferase activity. Minoxidil is a drug that exhibits hair growth effects via an ATP-sensitive potassium channel in a dermal papilla cell, and the medicinal effects of minoxidil are exhibited, when the minoxidil is converted to minoxidil sulfate by the action of sulfotransferase (minoxidil sulfotransferase) on the minoxidil in a living body. Accordingly, the jellyfish collagen reinforces the activity of minoxidil sulfotransferase in the scalp, so that it can enhance the hair growth effects of minoxidil.

### 2. Agent for regulating expression of hair growth/hair restoration-related factors

The above-described jellyfish collagen has an action to change (accelerate or reduce) the expression of hair growth/hair restoration-related factors in the scalp. Hence, the jellyfish collagen can also be used as an agent for regulating (promoting or suppressing) the expression of hair growth/hair restoration-related factors. In the present invention, the phrase "promotion or suppression of the expression of hair growth/hair restoration-related factors" is used to mean that the transcription of genes encoding hair growth/hair restoration-related factors into mRNA is promoted or suppressed, or the translation of the mRNA is promoted or suppressed, so that the expression levels of the hair growth/hair restoration-related factors can be significantly increased or decreased, compared with scalp onto which the jellyfish collagen is not applied. For example, the expression levels of hair growth/hair restoration-related factors in scalp, onto which the jellyfish collagen is applied, are desirably increased or decreased by 1.1 times or more, preferably 2 times or more, more preferably 3 times or more, and further preferably 5 times or more, compared with the expression levels thereof in scalp, onto which the jellyfish collagen is not applied.

The factors whose expression is accelerated by the jellyfish collagen include BMP2, BMPR1A, GSK3B, BCL2, VEGFB, VEGFC, FGF7, NOG, SMAD4, TGFB1, GAS1, THBS2, BMP7, EFNA1, IRF6, and SOX9.

BMP2 (Bone Morphogenetic Protein-2) is one type of bone morphogenetic protein. It has been known that if the function of BMP2 is inhibited in the skin, the growth of hair matrix cells in hair roots is abnormally increased and differentiation of hair is damaged. In addition, it has also been confirmed that the expression of BMP2 is particularly significantly reduced, among growth factor genes whose expression is reduced in male subjects with fallen hair (Recent Progression in Elucidation of Hair Growth Regulation Mechanism and Hair Restorers, Journal of Society Cosmetic Chemists Japan, 1999; 33: 220-228).

Regarding BMPR1A (Bone Morphogenetic Protein Receptor, type 1A), it has been demonstrated that a reduction in the expression level of a BMPR1A gene or a reduction in intracellular signals downstream thereof is likely to be one factor for the onset of fallen hair (BMPR1A signaling is necessary for hair follicle cycling and hair shaft differentiation in mice, Development 2004; 131: 1825-1833).

Regarding GSK3B (Glycogen Synthase Kinase 3 Beta), it has been reported that GSK3B is expressed in hair follicle stem cells and is associated with formation of hair roots (Expression and function of glycogen synthase kinase-3 in human hair follicles, Arch Dermatol Res. 2010; 302: 263-270).

Regarding BCL2 (B-cell CLL/lymphoma 2), it has been known that BCL2 suppresses the cell death (apoptosis) of dermal papilla cells (Hair follicle apoptosis and Bcl-2, J Investig Dermatol Symp Proc. 1999; 4: 272-277).

It has been reported that VEGFB (Vascular endothelial growth factor B) and VEGFC (Vascular endothelial growth factor C) form novel blood vessels to improve blood flow, have wound a healing promotion effect and a skin color improving effect, and further have hair restoring and/or hair nourishing effects, etc. In particular, with regard to the hair restoring and/or hair nourishing effects, VEGF has been identified as a main mediator for hair follicle growth and cycling. It has been known that, if generation of VEGF around hair follicles is increased, vascularization is promoted, and nutrients necessary for hair in the growth phase are efficiently supplied, leading to the achievement of hair restoration and/or hair nourishing promotion effects (Control of hair growth and follicle size by VEGF-mediated angiogenesis, J Clin Invest. 2001; 107: 409-417).

FGF7 (fibroblast growth factor 7) is also referred to as KGF (alias: keratinocyte growth factor), and is a multifunctional signal molecule exhibiting activities such as proliferation and differentiation on various cells such as fibroblasts. It has been known that FGF7 is generated from dermal papilla cells in the growth phase, acts on hair matrix cells, and promotes growth and division of the hair matrix cells, so that FGF7 allows the hair to grow (Japanese Patent No. 3578761).

NOG (Noggin) and SMAD4 (SMAD family member 4) are known as cellular signals necessary for induction of the hair cycle from a resting phase to a growth phase (Standardized Scalp Massage Results in Increased Hair Thickness by Inducing Stretching Forces to Dermal Papilla Cells in the Subcutaneous Tissue, Eplasty. 2016; 16: e8.).

It has been known that TGFBI (transforming growth factor, beta-induced), GAS1 (growth arrest specific 1), THBS2 (thrombospondin 2), BMP7 (bone morphogenetic protein 7), EFNA1 (ephrin A1), and IRF6 (interferon regulatory factor 6) are specifically expressed in dermal papilla cells having an ability to form and regenerate hair follicles (Patent Publication (Kokai) No. 2005-349081 A).

It has been reported that SOX9 (SRY (sex determining region Y)-box 9) is a transcriptional factor that plays an essential role in differentiation of chondrocytes, and that SOX9 supports the plasticity of stem cells in hair follicles (SOX9: a stem cell transcriptional regulator of secreted niche signaling factors, Genes Dev. 2014; 28: 328-341).

From each of the aforementioned findings, it is found that BMP2, BMPR1A, GSK3B, BCL2, VEGFB, VEGFC, FGF7, NOG, SMAD4, TGFB1, GAS1, THBS2, BMP7, EFNA1, IRF6, and SOX9 all function as hair growth-promoting factors, and that promotion of the expression of these factors is effective for hair growth and/or hair restoration.

On the other hand, the gene whose expression is decreased by the jellyfish collagen may be, for example, ILIA. ILIA (Interleukin-1 alpha) is an inflammatory factor, and ILIA has been known to have an action to suppress the growth of hair and an action to induce apoptosis (cell death) to hair. IL1A is considered to be a factor causing fallen hair, and it has been reported that ILIA is associated with the thinning hair of females (Association analysis of IL1A and IL1B variants in alopecia areata, Heredity (Edinb). 2001; 87: 215-219). As such, ILIA is a factor causing fallen hair, and suppression of the expression of ILIA is effective for hair growth and/or hair restoration.

### 3. Composition for scalp and hair

Since the jellyfish collagen has the above-described actions, the jellyfish collagen can be provided as a composition for scalp and hair that is formulated, together with minoxidil or a derivative thereof, into a preparation form suitable for use in scalp or hair. The composition for scalp and hair of the present invention can be processed into the form of a composition such as a cosmetic, a quasi-drug, or a pharmaceutical product. Specifically, the composition for scalp and hair of the present invention can be used as a therapeutic and/or preventive agent for diseases associated with the damage of scalp or hair (wherein the hair also includes eyebrows and eyelashes, as well as hair), such as thinning hair and alopecia, or as a hair care product having any one or two or more actions selected from a hair growth-promoting action, a hair-restoring action, a hair-nourishing action, and an alopecia (hair loss)-preventing action. The above-described alopecia includes androgenetic alopecia (AGA), fstrongale androgenetic alopecia (FAGA), female pattern hair loss (FPHL), alopecia areata, premature alopecia (alopecia of the middle age), post-illness/postpartum alopecia, diffuse alopecia, seborrheic alopecia, and alopecia pityrodes. Further, in the present invention, the term "hair growth and/or hair restoration" includes early induction of the growth phase in the hair cycle, extension of the growth phase, retardation of the regression phase, promotion of elongation of hair, and moisturization to scalp or hair.

Commercially available minoxidil can be used herein as minoxidil (chemical name: 2,4-diamino-6-piperidinopyrimidine-3-oxide). In addition, the minoxidil derivative may be, for example, pidioxidil (chemical name: 6-pyrrolidino-2,4-diaminopyrimidine-3 -oxide).

The amount of minoxidil or a derivative thereof mixed into the composition for scalp and hair of the present invention is preferably 0.01 to 20 (w/v) %, and more preferably 0.1 to 10 (w/v) %. In addition, the amount of the jellyfish collagen mixed into the present composition for scalp and hair is preferably 0.00001 to 10 (w/v) %, more preferably 0.0001 to 1 (w/v) %, and further preferably 0.001 to 0.1 (w/v) %. The above-described amounts are provided only as examples, and the mixed amounts may be determined and/or adjusted, as appropriate, while taking into consideration the type of the composition for scalp and hair (i.e., whether the composition is a pharmaceutical product or a hair care product, etc.), the form thereof, an applicable target, an application method, a common usage, effect-efficacy, costs, etc. Moreover, the content ratio between the jellyfish collagen and the minoxidil or a derivative thereof is, at a mass ratio, preferably 1 : 10 to 1 : 10000, and more preferably 1 : 100 to 1 : 1000, in order to exhibit the effects of the combined use.

In the present invention, the composition for scalp and hair broadly indicates those used for scalp or hair, and the present composition for scalp and hair is not particularly limited as long as it is applicable to scalp or hair. The dosage form thereof is not particularly limited. The dosage form of the present composition for scalp and hair may be any of liquid, gel, emulsion, cream, paste, and spray. The specific product form of the present composition for scalp and hair is preferably an external preparation such as hair lotion, hair tonic, scalp essence, hair cream, hair gel, hair shampoo, hair rinse, hair conditioner, hair spray, or a hair pack, and is particularly preferably an external liquid preparation such as hair lotion, hair tonic, or scalp essence.

The composition for scalp and hair of the present invention can be produced by appropriately selecting and mixing base agents, additives, various types of components, and the like, which are commonly used in a skin external composition, depending on the type, dosage form and shape thereof, etc., and then, by applying an ordinary method. Examples of the base agents, additives and various types of components may include: dissolving agents (purified water, ethanol, denatured ethanol, etc.), thickeners (ethyl cellulose, carboxymethyl cellulose, a carboxyvinyl polymer, xanthan gum, methyl cellulose, gelatin, etc.), polyhydric alcohols (myristyl alcohol, cetanol, stearyl alcohol, behenyl alcohol, glycerin, propylene glycol, 1,3-butylene glycol, etc.), surfactants (sorbitan fatty acid ester, polyglycerin fatty acid ester, polyoxyethylene sorbitan fatty acid ester, polyoxyethylene sorbit fatty acid ester, polyoxyethylene glycerin fatty acid ester, polyethylene glycol fatty acid ester, polyoxyethylene alkyl ether, polyoxyethylene polyoxypropylene alkyl ether, polyoxyethylene alkyl phenyl ether, polyoxyethylene hardened castor oil, etc.), oils and fats (olive oil, coconut oil, evening primrose oil, jojoba oil, castor oil, hardened castor oil, etc.), waxes (lanolin, beeswax, carnauba wax, etc.), hydrocarbons (liquid paraffin, squalene, squalane, petrolatum, etc.), fatty acids (lauric acid, myristic acid, palmitic acid, stearic acid, etc.), esters (isopropyl myristate, isopropyl palmitate, cetyl octanoate, glycerin trioctanoate, octyldodecyl myristate, octyl stearate, stearyl stearate, etc.), organic acids (citric acid, lactic acid, α-hydroxyacetic acid, pyrrolidone carboxylic acid, etc.), sugars (maltitol, sorbitol, N-acetyl-D-glucosamine, etc.), antioxidants (tocopherol acetate, ascorbic acid, butylated hydroxytoluene, sodium bisulfite, benzotriazole, etc.), vasodilators (carpronium chloride, benzyl nicotinate, Swertia japonica extract, Panax ginseng extract, capsicum tincture, etc.), antihistamines (diphenhydramine hydrochloride, isothipendyl hydrochloride, etc.), antiinflammatory agents (glycyrrhetinic acid, glycyrrhizinic acid, tranexamic acid, guaiazulene, etc.), ultraviolet protection agents (octyl methoxycinnamate, oxybenzone, urocanic acid, etc.), keratolytic agents (urea, salicylic acid, resorcin, etc.), transdermal absorption promoting agents (polyalkylene glycols such as polyethylene glycol or polypropylene glycol, fatty acid esters such as ethyl oleate or octyldodecyl lactate, etc.), refreshing agent (menthol, menthyl glyceryl ether, camphor, borneol, cineolmenton, methyl salicylate, spearmint oil, peppermint oil, mint oil, eucalyptus oil, etc.), moisturizers (sodium hyaluronate, chondroitin sulfate, etc.), disinfectants (chlorhexidine gluconate, isopropylmethylphenol, quaternary ammonium salt, hinokitiol, piroctone olamine, etc.), amino acids (pantothenic acid and a derivative thereof, glycyrrhizic acid and a derivative thereof, nicotinic acid ester, serine, methionine, etc.), vitamins (vitamin A, vitamin B₆, vitamin E and a derivative thereof, biotin, etc.), preservatives (methyl paraoxybenzoate, propyl paraoxybenzoate, phenoxyethanol, etc.), perfumes, and coloring agents. The above-described base agents, additives, and various types of components may be mixed each alone (each only one type), or may be used in combination of two or more types. Moreover, the base agents, additives, and various types of components may also be used in combination with other drugs or plant extracts having hair restoring and/or hair nourishing effects, within a range that does not impair the effects of the present invention.

The composition for scalp and hair of the present invention can be used, for example, by being applied or sprayed, in an appropriate amount, onto scalp or hair, once or several times a day. Accordingly, the present invention also relates to a beauty treatment method, which comprises applying the above-described composition for scalp and hair to scalp or hair. In the present invention, the beauty treatment method does not include medical treatments to humans (therapies and treatments that are approved to be carried out only by doctors or by healthcare professionals who receive doctor's instructions, such as nurses and/or midwifes, according to the Medical Act). Moreover, the beauty treatment method does not only include the case of applying the method just personally, but also includes a case where the method is applied to other persons. When a product is provided, sales staff, estheticians and other people may also perform the present beauty treatment method.

### Examples

Hereinafter, the present invention will be more specifically described in the following examples. However, these examples are not intended to limit the scope of the present invention.

### (Reference Example) Preparation of jellyfish-derived collagen (roughly purified)

A jellyfish (moon jellyfish)-derived collagen used in the following Examples was prepared by the following method.
1) A body of jellyfish that was in a refrigerated state was washed with water, and was then separated into a solid and a liquid, using a strainer.
2) The separated solid was crushed using a crusher. A fragment obtained by crushing was used as a sample.
3) The sample obtained in 2) above and water were placed in an extraction stirring tank, and were then subjected to stirring and extraction at 4°C.
4) An aqueous solution obtained by the stirring and extraction in 3) above was centrifuged also at 4°C, at 10000 G for 10 minutes to obtain a liquid component.
5) Isopropyl alcohol was added into the liquid component obtained in 4) above to result in an isopropyl alcohol concentration of 70% by volume, and as a result, a gelatinous precipitate was generated.
6) A solution containing the gelatinous precipitate obtained in 5) above was stirred overnight while the temperature was kept at 4°C, and thereafter, the reaction solution was centrifuged also at 4°C, at 10000 G for 10 minutes to obtain a solid.
7) An aqueous solution of isopropyl alcohol in a concentration of 60% by mass that was 3 times the mass of the solid obtained in 6) above was added to the solid.
8) The mixture generated in 7) above was stirred at 4°C for 5 minutes, and was then centrifuged also at 4°C at 10000 G for 10 minutes to obtain a solid.
9) The solid obtained in 8) above was subjected to the washing operations in 7) and 8) above again.

As described above, a protein separation step was terminated.
10) An aqueous solution of isopropyl alcohol in a concentration of 25% by mass that was 3 times the mass of the solid obtained in 9) above was added to the solid.
11) The solution obtained in 10) above was stirred at 4°C for 5 minutes, and was then centrifuged also at 4°C at 10000 G for 10 minutes to obtain a solid.
12) The solid obtained in 11) above was further subjected to the operations in 10) and 11) above twice, so that the solid was separated from a liquid component.
13) The solid obtained in 12) above was suspended in water, and was then subjected to a dialysis treatment and freeze-drying, so as to obtain a final solid (collagen).

### (Example 1) Analysis of expression of minoxidil sulfotransferase genes

### (1) Method

An epidermal keratinocyte line (PHK16-0b; obtained from JCRB Cell Bank; JCRB0141) was seeded on a petri dish to a cell density of 20 × 10⁴ cells/mL. The cells were cultured for 1 week in a CO₂ incubator (5% CO₂, 37°C) in an MCDB153 medium supplemented with 5 µg/mL insulin, 0.5 µg/mL hydrocortisone, 10 µg/mL transferrin, 14.1 µg/mL ethanolamine phosphate, 6 ng/mL ethanolamine, 40 µg/mL bovine pituitary extract (BPE), and 10 ng/mL epidermal growth factor (EGF), while the medium was exchanged with a fresh one, once every 2 or 3 days. After completion of the culture, the medium was exchanged with an MCDB153 medium, to which the jellyfish collagen (solid) prepared in the above Reference Example was added to a concentration of 30 µg/mL (a jellyfish collagen-added medium), and the resulting cells were further cultured for 2 weeks, while the medium was exchanged with a fresh one, once every 2 or 3 days. At the same time, a sample, which was cultured in the same MCDB153 medium as described above, was also prepared (a control). After completion of the culture, total RNA was extracted from the cells, using Rnase Mini kit. This total RNA was subjected to GeneChip analysis using GeneChip Gene Analysis System manufactured by Affimetry.

A non-addition group was used as a control, a gene group (up) and a gene group (down), in which the expression signals were above or below the detection limit, and the expression signals were increased or decreased, respectively, in a sample (a jellyfish collagen solution) addition group, compared with the expression signals of the control, were extracted, and were each subjected to a gene ontology (GO) analysis.

The results of the expression analysis of sulfotransferase genes are shown in Table 1.

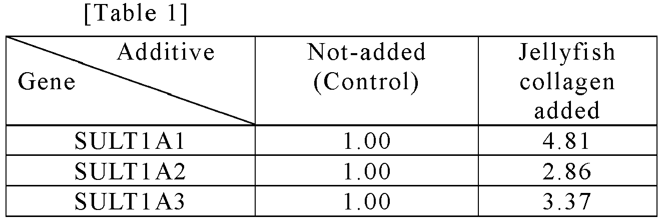

As shown in Table 1, the expression levels of the minoxidil sulfotransferase genes, namely, SULT1A1 (sulfotransferase family, cytosolic, 1A, phenol-preferring, member 1), SULT1A2 (sulfotransferase family, cytosolic, 1A,phenol-preferring, member 2), and SULT1A3 (sulfotransferase family, cytosolic, 1A, phenol-preferring, member 3) were all significantly increased in epidermal cells to which the jellyfish collagen had been added, compared with in epidermal cells to which the jellyfish collagen had not been added.

### (Example 2) Analysis of expression of hair growth/hair restoration-related genes

The expression of hair growth/hair restoration-related genes was analyzed in the same manner as that of Example 1. The results are shown in Table 2.

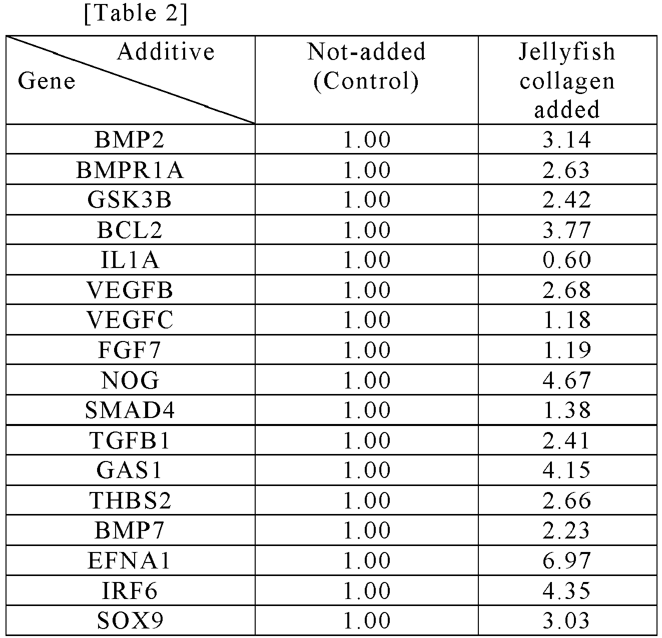

As shown in Table 2, the expression levels of the expression-promoting factors BMP2, BMPR1A, GSK3B, BCL2, VEGFB, VEGFC, FGF7, NOG, SMAD4, TGFB1, GAS1, THBS2, BMP7, EFNA1, IRF6, and SOX9 were significantly increased in epidermal cells to which the jellyfish collagen had been added, compared with in epidermal cells to which the jellyfish collagen had not been added. In contrast, the expression level of the hair loss factor IL1A was significantly decreased in epidermal cells to which the jellyfish collagen had been added, compared with in epidermal cells to which the jellyfish collagen had not been added.

### Industrial Applicability

The present invention can be utilized in the field of manufacturing cosmetics, quasi-drugs, and pharmaceutical products, which are capable of improving and/or preventing thinning hair and fallen hair, and promoting hair growth.

All publications, patents and patent applications cited in the present description are incorporated herein by reference in their entireties.

## Claims

1. A minoxidil sulfotransferase activity enhancer, comprising a jellyfish collagen as an active ingredient.

2. An agent for promoting the expression of one or two or more types of hair growth/hair restoration-related factors selected from the group consisting of BMP2, BMPR1A, GSK3B, BCL2, VEGFB, VEGFC, FGF7, NOG, SMAD4, TGFB1, GAS1, THBS2, BMP7, EFNA1, IRF6, and SOX9, wherein the agent comprises a jellyfish collagen as an active ingredient.

3. An agent for suppressing the expression of IL1A, comprising a jellyfish collagen as an active ingredient.

4. A composition for scalp and hair, comprising a jellyfish collagen and minoxidil or a derivative thereof.

5. The composition for scalp and hair according to claim 4, wherein the content ratio between the jellyfish collagen and the minoxidil or a derivative thereof is 1 : 10 to 1 : 10000 at a mass ratio.

6. The composition for scalp and hair according to claim 4 or 5, which is in the form of a cosmetic, a quasi-drug, or a pharmaceutical product

7. A beauty treatment method, comprising applying the composition for scalp and hair according to any one of claims 4 to 6 to scalp or hair.
